Europäisches Patentamt

European Patent Office

Office européen des brevets

(11) Veröffentlichungsnummer: **0 442 516 A1**

# EUROPÄISCHE PATENTANMELDUNG

(21) Anmeldenummer: **91102159.0**

(22) Anmeldetag: **15.02.91**

(51) Int. Cl.5: **F16D 41/08**, A61M 5/145, A61M 3/00

(30) Priorität: **16.02.90 DE 4004925**

(43) Veröffentlichungstag der Anmeldung:
**21.08.91 Patentblatt 91/34**

(84) Benannte Vertragsstaaten:
**AT CH DE FR GB IT LI NL**

(71) Anmelder: **MGVG MEDIZINISCHE GERÄTE
VERTRIEBS GMBH**
**Hans-Pinsel-Strasse 10B**
**W-8013 Haar(DE)**

(72) Erfinder: **Döring, Jochen, c/o MGVG GmbH,**
**Niederlassung**
**Nordrhein-Westfalen, Quettinger Strasse 210**
**W-5090 Leverkusen(DE)**

(74) Vertreter: **Vossius & Partner**
**Siebertstrasse 4 P.O. Box 86 07 67**
**W-8000 München 86(DE)**

(54) **Schaltbarer Freilauf.**

(57) Ein schaltbarer Freilauf für eine Kupplungseinrichtung, insbesondere in einer Infusionsspritzenpumpe, weist eine in einer Führung (4) drehbare
Welle (1) mit an der Führung anliegenden beweglichen Klemmelementen (6) auf, die die Drehung der
Welle in eine Richtung blockieren, wobei sich die
Klemmelemente zwischen der Welle und der Führung verkeilen. Ein zur Welle konzentrischer ringförmiger Schaltkranz (5) ist vorgesehen, der das Blok-
kieren der Drehbewegung der Welle verhindert, so
daß diese in beide Richtungen drehbar ist.

Die erfindungsgemäße Ausführungsform ermöglicht eine einfache und zuverlässige Bedienung des
schaltbaren Freilaufs.

Fig. 2

Die Erfindung betrifft einen schaltbaren Freilauf für eine Kupplungseinrichtung, die insbesondere in einer Infusionsspritzenpumpe Verwendung finden kann.

Ein derartiger schaltbarer Freilauf wird z.B. im Zusammenhang mit dem Aus- und Einbau der Infusionsspritze und dem Füllen der Spritze mit einem Injektat benutzt. Nach dem Füllen der Spritze ist es zur Vermeidung von schädlichen Folgen für den Patienten erforderlich, daß die Flüssigkeit genau das vordere Ende eines mit der Spritze verbundenen Schlauchleiters erreicht. Das geschieht im Stand der Technik durch ein entsprechendes Verschieben des Kolbens über den mit ihm verbundenen Schieber der Infusionsspritzenpumpe. Zur Vermeidung einer Fehlbedienung darf diese Verschiebung nur in eine Richtung (Kolbenbewegung in Richtung auf den Spritzenausgang) erfolgen. Die Verschiebung des Schiebers erfolgt durch die Drehbewegung einer Spindel an der Infusionsspritzenpumpe, wobei eine Drehung in entgegengesetzter Richtung durch Klemmelemente blockiert wird. Es wird somit eine Kupplung zwischen einer Drehvorrichtung und der Antriebsspindel der Infusionsspritzenpumpe ausgebildet, die einen Freilauf aufweist. Beim Ein- und Ausbau der Infusionsspritze oder z.B. bei Wartungsarbeiten an der Infusionsspritzenpumpe muß die Spindel aus dem Pumpengehäuse herausgedreht werden. Zu diesem Zweck muß die blockierte Drehrichtung des Freilaufs gängig gemacht werden. Das geschieht durch einen schaltbaren Freilauf.

Aus der DE-31 50 623 ist ein schaltbarer Freilauf bekannt. Dieser weist einen Schaltring auf, der aus einem Käfig mit zwei radial entgegengesetzt angeordneten Nadeln besteht und auf der mit der Spindel verbundenen Drehwelle drehbar ist. Die Nadeln liegen an einem mit der Spindel verbundenen Wellenabschnitt außen an. Weiterhin sind an dem Schaltring tangential anliegende Druckstücke vorgesehen. Diese bilden mit der Drehwelle einen keilförmigen Zwischenraum, der bewirkt, daß sich der Käfig mit den Nadeln in einer Richtung verkeilt und in der anderen frei beweglich ist. Dadurch läßt sich die Drehwelle nur in eine Richtung frei drehen und es entsteht eine Freilaufwirkung. Tangential zum Schaltring ist ferner ein Druckstift, der gegen eine Feder gedrückt wird, angeordnet. Dieser ist über eine Nase des Schaltrings und eine Querkerbe mit dem Schaltring in Eingriff und schiebt beim Drücken gegen die Feder den Schaltring in die Freigabeposition des Freilaufs, so daß die mit der Spindel verbundene Drehwelle in beide Drehrichtungen gedreht werden kann.

Ein Nachteil des bekannten schaltbaren Freilaufs besteht darin, daß beim Herausziehen des Schiebers, bei dem sich die Spindel in die freigegebene Richtung dreht, ständig mit der Hand der Knopf gedrückt werden muß. Außerdem ist die Anordnung des Stiftes der Feder und der entsprechenden Führung mit einem beträchtlichen konstruktiven Aufwand verbunden.

Der Erfindung liegt die Aufgabe zugrunde, einen schaltbaren Freilauf zur Verfügung zu stellen, der manuell leicht zu bedienen ist und besonders einfach und kostengünstig herzustellen ist.

Die Lösung dieser Aufgabe wird mit den Merkmalen der Patentansprüche erreicht.

Bei dieser Lösung liegt der Erfindung der Gedanke zugrunde, die Schaltung des Freilaufs nicht tangential zu einem Schaltring anzuordnen, wie das im Stand der Technik vorgesehen ist, sondern das Schaltelement wird konzentrisch zur Drehwelle als Schaltkranz angeordnet.

Diese Lösung ist als besonders vorteilhaft anzusehen, da sie eine einfache Bedienung, z.B. bei einer erfindungsgemäßen Ausführungsform durch ruckartiges Bewegen der Drehwelle, und eine kostengünstige Herstellung ermöglicht.

Die Erfindung wird nachstehend mit Bezug auf die Zeichnungen näher erläutert. Es zeigen:

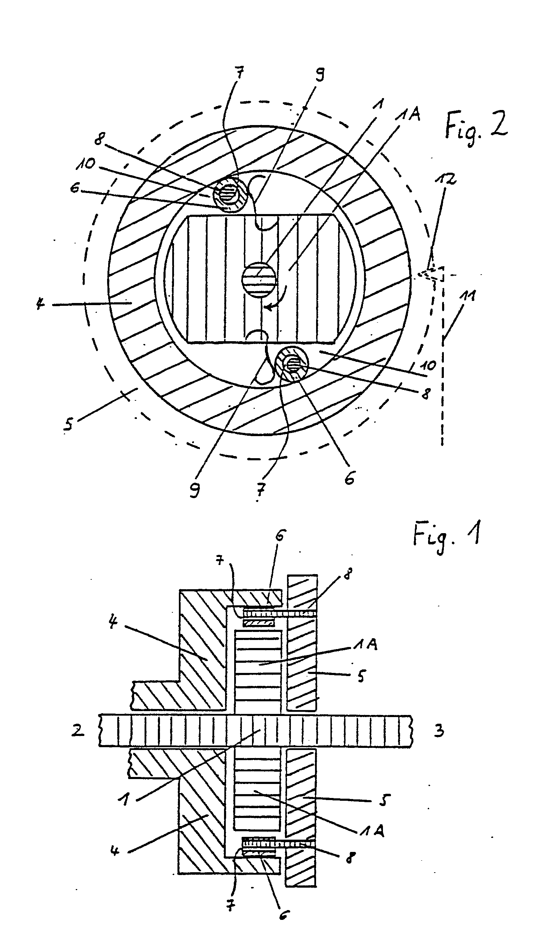

Figur 1    Querschnitt eines schaltbaren Freilaufs in Längsausdehnung der Drehwelle, und

Figur 2    Querschnitt eines schaltbaren Freilaufs senkrecht zur Längsausdehnung der Drehwelle.

Eine erfindungsgemäße Ausführungsform des schaltbaren Freilauf weist eine Welle 1 auf, die antriebsseitig eine Spindel 2 und am anderen Ende eine Drehvorrichtung 3 aufweist. Die Welle 1 wird mit einem Wellenstück 1A in einem Gehäuse 4 geführt. Zwischen dem Gehäuse 4 und dem Wellenstück 1A sind durch entsprechende Aussparungen des Wellenstücks 1A zwei Zwischenräume 10 ausgebildet, in denen zwei Walzen 6 mit kreisförmigen zentralen Bohrungen 7 beweglich sind. Auf der Welle 1 ist gegen diese drehbar ein Schaltkranz 5 angeordnet. Dieser weist zwei Bolzen 8 auf, die mit den kreisförmigen Öffnungen 7 der zwei Walzen 6 in Eingriff stehen. An dem Wellenstück 1A sind zwei Federn 9 angeordnet, die für die Walzen 6 einen Anschlag bilden und diese in Richtung der Verengung des keilförmigen Zwischenraumes 10 zwischen dem Wellenstück 1A und der Führung 4 drücken. Im nicht-geschalteten Zustand ist die Welle 1 nur im Uhrzeigersinn drehbar. Bei einer Drehung der Welle 1 im Gegenuhrzeigersinn werden die Walzen 6 und der mit ihm verbundene Schaltkranz 5 durch die Federn 9 und das Wellenstück 1A im Gegenuhrzeigersinn um die Welle 1 gedreht, so daß sie in die keilförmige Verengung des Zwischenraums 10 geraten und so eine weitere Drehbewegung blockieren. Durch eine Drehung des Schaltkranzes 5 im Uhrzeigersinn um die Welle 1 werden die Walzen 6 aus der Verkeilung befreit und eine Drehung der Welle 1 im Gegenuhrzeigersinn freigegeben. Die Drehung des Schaltkranzes 5 um die Welle 1 erfolgt gegen die Kraft der Federn 9, so daß beim Wegfall der Kraftwirkung auf den Schaltkranz 5 die Walzen 6 in die Blockierstellung des Freilaufs zurückgleiten.

Eine andere erfindungsgemäße Ausführungsform sieht vor, daß der periphere Teil des Schaltkranzes 5 ein höheres Gewicht als der zentrale Teil aufweist. Dadurch tritt ein erhöhtes Drehmoment bezüglich der Drehwelle 1 auf, und der Schaltkranz 5 läßt sich aufgrund seiner Trägheit durch ruckartiges Drehen der Drehwelle 1 im Uhrzeigersinn in die Freigabeposition des Freilaufes schalten.

Eine besondere erfindungsgemäße Ausführungsform sieht einen elastischen Anschlag 11 vor, der mit der Welle 1 verbunden ist und in eine Kerbe 12 des Schaltkranzes 5 in der Freigabestellung des Freilaufes einrastet und so ein Zurückgleiten der Walzen 6 in die Blockierstellung des Freilaufes verhindert.

**Patentansprüche**

1. Schaltbarer Freilauf für eine Kupplungseinrichtung, insbesondere in einer Infusionsspritzenpumpe, mit einer in einer Führung (4) drehbaren Welle (1) mit einem Wellenstück (1A), an der Führung (4) anliegenden, beweglichen Klemmelementen (6), die die Drehung der Welle (1) in eine Richtung blockieren, wobei sich die Klemmelemente (6) zwischen dem Wellenstück (1A) und der Führung (4) verkeilen und mit einer Schaltung, die wahlweise das Blockieren der Drehbewegung der Welle (1) verhindert, so daß diese in beide Richtungen drehbar ist, dadurch gekennzeichnet,
   (a) daß die Klemmelemente aus zwei Walzen (6) bestehen, die innerhalb eines keilförmigen Zwischenraumes (10), der zwischen dem Wellenstück (1A), der Führung (4) und einem Anschlag (9) ausgebildet wird, beweglich sind, und
   (b) daß ein zur Welle (1) konzentrischer, auf der Welle drehbarer Schaltkranz (5) als Schaltung vorgesehen ist, der in einer zu den Walzenquerschnittsflächen parallelen Ebene angeordnet ist und mit den Walzen (6) in Eingriff steht, wobei durch eine entsprechende Drehung bzw. Stellung des Schaltkranzes (5) die Verkeilung der Klemmelemente (6) und damit die Blockierung des Freilaufs aufgehoben bzw. verhindert wird.

2. Schaltbarer Freilauf nach Anspruch 1, dadurch gekennzeichnet, daß der Eingriff zwischen den Walzen (6) und dem Schaltkranz (5) mechanisch erfolgt.

3. Schaltbarer Freilauf nach Anspruch 1, dadurch gekennzichnet, daß der Eingriff zwischen den Walzen (6) und dem Schaltkranz (5) durch magnetische Anziehung erfolgt.

4. Schaltbarer Freilauf nach einem der Ansprüche 1 bis 3, dadurch gekennzeichnet, daß der Schaltkranz (5) so ausgebildet ist, daß bei einer ruckartigen Bewegung der Welle (1) entgegengesetzt zur blockierten Richtung ein entsprechend hohes Drehmoment auftritt, so daß sich der Schaltkranz (5) auf der Welle (1) dreht und die Blockierung gelöst wird.

5. Schaltbarer Freilauf nach Anspruch 1, 2 oder 4, dadurch gekennzeichnet,
   (a) daß die Walzen (6) zentrische kreisförmige Bohrungen (7) aufweisen,
   (b) daß zwei sich radial gegenüberstehende Bolzen (8) in der den Walzen (6) gegenüberliegenden Ringfläche des Schaltkranzes angeordnet sind und mit den Bohrungen (7) der Walzen (6) drehbar in Eingriff stehen, und
   (c) daß der Anschlag (9) der Walzen durch zwei Federn ausgebildet wird, die die Walzen (6) in Richtung auf die Verengung des keilförmigen Zwischenraums (10) drücken.

6. Schaltbarer Freilauf nach einem der Ansprüche 1 bis 5, dadurch gekennzeichnet, daß in der Freigabestellung des Freilaufs eine Einrastung des Schaltkranzes (5) mittels eines elastischen Eingriffs (11, 12) in die äußere Oberfläche des Schaltkranzes (5) erfolgt.

Fig. 2

Fig. 1

**Europäisches Patentamt**

# EUROPÄISCHER RECHERCHENBERICHT

Nummer der Anmeldung

**EP 91 10 2159**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | GB-A-1 212 86 (PUMP SEPARATOR)<br>* Seite 2, Zeile 6 - Zeile 29; Abbildung 1 *<br>- - - | 1 | F 16 D 41/08<br>A 61 M 5/145<br>A 61 M 3/00 |
| A | US-A-3 476 226 (MASSEY)<br>* ZUSAMMENFASSUNG *<br>- - - | 1 | |
| A | US-A-1 550 996 (WILCOX)<br>* Seite 2, Zeile 52 - Zeile 61; Abbildung 4 *<br>- - - | 1 | |
| A,D | DE-A-3 150 623 (BRAUN MELSUNGEN)<br>- - - - - | 1 | |

| | RECHERCHIERTE SACHGEBIETE (Int. Cl.5) |
|---|---|
| | A 61 M<br>F 16 D |

Der vorliegende Recherchenbericht wurde für alle Patentansprüche erstellt

| Recherchenort | Abschlußdatum der Recherche | Prüfer |
|---|---|---|
| Den Haag | 21 Mai 91 | VLECK J.M. |